Europäisches Patentamt

(19) European Patent Office

Office européen des brevets

(11) Veröffentlichungsnummer : **0 248 863 B1**

# (12) EUROPÄISCHE PATENTSCHRIFT

(45) Veröffentlichungstag der Patentschrift :
**20.02.91 Patentblatt 91/08**

(51) Int. Cl.$^5$ : **A61K 33/16,** // (A61K33/16,
**31:19, 31:05)**

(21) Anmeldenummer : **87900120.4**

(22) Anmeldetag : **11.12.86**

(86) Internationale Anmeldenummer :
**PCT/EP86/00737**

(87) Internationale Veröffentlichungsnummer :
**WO 87/03482 18.06.87 Gazette 87/13**

(54) **PHARMAZEUTISCHES PRODUKT ENTHALTEND EIN GEMISCH AUS BENZOESÄURE, PHENOL UND EIN ALKALIFLUORID ZUR TOPISCHEN BEHANDLUNG VON HERPES-VIRUS-EFFLUORESZENZEN.**

Verbunden mit 86117298.9/0226187
(europäische
Anmeldenummer/Veröffentlichungsnummer)
durch Entscheidung vom 31.07.89.

(30) Priorität : **12.12.85 DE 3543801**

(43) Veröffentlichungstag der Anmeldung :
**16.12.87 Patentblatt 87/51**

(45) Bekanntmachung des Hinweises auf die
Patenterteilung :
**20.02.91 Patentblatt 91/08**

(84) Benannte Vertragsstaaten :
**AT BE CH DE ES FR GB GR IT LI LU NL SE**

(56) Entgegenhaltungen :
**Dictionnaire Vidal, 56. Ausgabe, 1980, OVP,
(Paris, FR), siehe Seite 478: "Fluocaril Bi-
Fluoré bain de bouche", und "Fluocaril Bi-
Fluoré gel dentaire"**

(73) Patentinhaber : **Marquardt, Bernd Dr.
Auf der Heide 1c
D-4040 Neuss (DE)**

(72) Erfinder : **Marquardt, Bernd Dr.
Auf der Heide 1c
D-4040 Neuss (DE)**

(74) Vertreter : **Patentanwaltsbüro Cohausz &
Florack
Schumannstrasse 97
D-4000 Düsseldorf 1 (DE)**

## Beschreibung

Die vorliegende Erfindung betrifft ein neuartiges pharmazeutisches Produkt enthaltend ein Gemisch aus Benzoesäure, Phenol und einem Alkalifluorid in wässriger oder wässrig-alkoholischer Lösung, gegebenenfalls in Anwesenheit anderer üblicher Zusatzstoffe zu pharmazeutischen Zubereitungen in flüssiger oder halbflüssiger Form, und seine Verwendung zur Bekämpfung von durch Herpes-Virus bedingten Erkrankungen am Menschen.

Durch Herpes-Virus bedingte Erkrankungen des Menschen bewirken im allgemeinen mehr oder weniger starke Blasenbildung, insbesondere auf Schleimhäuten beim Menschen die Bildung von zum Teil sehr schmerzenden oder stark juckenden Blasen. Eine besonders bekannte Form derartiger Erkrankungen ist Herpes simplex.

Ein Produkt, das sowohl Benzoesäure als auch Phenol und Natriumfluorid enthält, ist auf dem Markt und wird in stark mit Wasser verdünntem Zustand als Desinfektionsmittel für den Mundraum eingesetzt (s. z.B. DICTIONNAIRE VIDAL, 56. Auflage, 1980, OVP (Paris, FR) S. 478). Dem Gegenstand der Erfindung entgegenstehende neuheitsschädliche Entgegenhaltungen sind dem Erfinder unbekannt.

Überraschenderweise sind Gemische aus Benzoesäure, Phenol und einem Alkalifluorid, vorzugsweise Natriumfluorid, in wässriger oder wässrig-alkoholischer Lösung, die gegebenenfalls andere übliche Zusatzstoffe zu pharmazeutischen Zubereitungen in flüssiger oder halbflüssiger Form enthalten, in nicht stark mit Wasser verdünnter Form zu einer raschen und wirkungsvollen Bekämpfung von durch Herpes-Virus bedingten Erkrankungen am Menschen, insbesondere zu äußerlichen Behandlung der erkrankten Hautpartien, insbesondere Schleimhautpartien geeignet.

Die einzelnen Bestandteile sind in zahlreichen pharmazeutischen Zubereitungen bekannt. So werden Benzoesäure und Phenol als Antiseptikum eingesetzt und Natriumfluorid wird zur Kariesprophylaxe eingesetzt.

Das erfindungsgemäße pharmazeutische Produkt zur Bekämpfung von durch Herpes-Viren bedingte Erkrankungen am Menschen ist dadurch gekennzeichnet, daß es neben Wasser oder Wasser-Alkohol als Lösungsmittel und neben üblichen Zusatzstoffen zu pharmazeutischen Zubereitungen in flüssiger Form oder halbflüssiger Form auf 100 g Endprodukt 0,1-0,5 g des Alkalifluorids, insbesondere Natriumfluorid, enthält, bevorzugt 0,2-0,4 g Benzoesäure, 0,6-0,8 g Phenol und 0,2-0,4 g des Alkalifluorids.

### Beispiel 1

100 g einer wässrigen Lösung enthalten 0,65 g einer 1 : 5-Lösung von Benzoesäure in Äthylalkohol, 0,73 g Phenol und 0,22 g Natriumfluorid. Das Produkt enthält zusätzlich 5,88 g Mentholöl, 1,2 g 01 Carbophylli, 1,63 g Salizylsäuremethylester und 0,74 g p-Hydroxybenzoesäureäthylester.

Die Bestandteile werden innig vermischt und in Behälter mit einem solchen Verschluß gegeben, mittels dessen das Produkt tropfenweise auf die befallenen Stellen aufgetupft werden kann.

### Beispiel 2

100 g einer wässrigen Lösung enthalten 2,4 g einer 1 : 5-Lösung von Benzoesäure in Äthylalkohol (0,4 g Benzoesäure und 2,0 g 96%-igem Alkohol), 1 g Phenol und 0,4 g Natriumfluorid. Sie enthält keine Zuzatzstoffe z.B. zur Verbesserung des Geruchs. Die Bestandteile werden wie im Beispiel 1 verarbeitet.

## Ansprüche

1. Pharmazeutisches Produkt enthaltend ein Gemisch aus Benzoesäure, Phenol und einem Alkalifluorid, in wässriger oder wässrig-alkoholischer Lösung, gegebenenfalls in Anwesenheit anderer üblicher Zusatzstoffe zu pharmazeutischen Zubereitungen zur topischen Behandlung von Herpes-Virus-Effluoreszenzen, dadurch gekennzeichnet, daß das Produkt auf 100 g 0,1 bis 0,5 g Benzoesäure, 0,5 bis 1,0 g Phenol und 0,1 bis 0,5 g des Alkalifluorids enthält.

2. Pharmazeutisches Produkt gemäß Anspruch 1, dadurch gekennzeichnet, daß es auf 100 g 0,2 bis 0,4 g Benzoesäure, 0,6 bis 0,8 g Phenol und 0,2 bis 0,4 g des Alkalifluorids enthält.

3. Pharmazeutisches Produkt gemäß Anspruch 1 oder 2, dadurch gekennzeichnet, daß das Alkalifluorid Natriumfluorid ist.

4. Verwendung einer wässrigen oder wässrigalkoholischen Lösung, die auf 100 g 0,1 bis 0,5 g Benzoesäure, 0,5 bis 1,0 g Phenol und 0,1 bis 0,5 g eines Alkalifluorids und gebebenenfalls andere übliche Zusatzstoffe enthält, zur Herstellung von pharmazeutischen zubereitungen zur topischen Behandlung von Herpes-Virus-Effluoreszenzen.

5. Verwendung gemäß Anspruch 4, dadurch gekennzeichnet, daß das verwendete Produkt auf 100 g 0,2 bis 0,4 g des Alxalifluorids enthält.

6. Verwendung gemäß Anspruch 4, dadurch gekennzeichnet, daß das Alkalifluorid Natriumfluorid ist.

## Patentansprüche für Vertragsstaat AT

1. Verfahren zur Herstellung von pharmazeutischen Produkten zur topischen Behandlung von Herpes-Virus-Effluoreszensen, dadurch gekennzeichnet, daß man Benzoesäure, Phenol und ein Alkalifluorid in Wasser oder einem Gemisch aus Wasser und Alkohol in üblicher Weise in solchen Mengen löst, daß das Produkt auf 100 g 0,1 bis 0,5 g Benzoesäure, 0,5 bis 1,0 g Phenol und 0,1 bis 0,5 g des Alkalifluorids enthält.

2. Verfahren gemäß Anspruch 1, dadurch gekennzeichnet, daß man Benzoesäure, Phenol und das Alkalifluorid in solchen Mengen löst, daß das resultierende Produkt auf 100 g 0,2 bis 0,4 g Benzoesäure, 0,6 bis 0,8 g Phenol und 0,2 bis 0,4 g des Alkalifluorids enthält.

3. Verfahren gemäß Anspruch 1 oder 2, dadurch gekennzeichnet, daß das Alkalifluorid Natriumfluorid ist.

4. Verwendung einer wässrigen oder wässrigalkoholischen Lösung, die auf 100 g 0,1 bis 0,5 g Benzoesäure, 0,5 bis 1,0 g Phenol und 0,1 bis 0,5 g eines Alkalifluorids und gegebenenefalls andere übliche Zuatzstoffe enthält, zur Herstellung von pharmazeutischen Zubereitungen zur topischen Behandlung von Herpes-Virus-Effluoreszenzen.

5. Verwendung gemäß Anspruch 4, dadurch gekennzeichnet, daß das verwendete Produkt auf 100 g 0,2 bis 0,4 g des Alkalifluorids enthält.

6. Verwendung gemäß Anspruch 4, dadurch gekennzeichnet, daß das Alkalifluorid Natriumfluorid ist.

## Claims

1. A pharmaceutical product containing a mixture of benzoic acid, phenol and an alkali fluoride, in an aqueous or aqueous-alcoholic solution, optionally in the presence of other usual additives for pharmaceutical preparations, for the topical treatment of herpes virus efflorescenes, characterized in that 100 g of the product contain from 0.1 to 0.5 g of benzoic acid, from 0.5 to 1.0 g of phenol and from 0.1 to 0.5 g of the alkali fluoride.

2. A pharmaceutical product as defined by claim 1, characterized in that 100 g of the product contains from 0.2 to 0.4 g of benzoic acid, 0.6 to 0.8 g of phenol and 0.2 to 0.4 g of the alkali fluoride.

3. A pharmaceutical product as defined by claim 1, characterized in that the alkali fluoride is sodium fluoride.

4. The use of an aqueous or aqueous-alcoholic solution containing per 100 g thereof from 0.1 to 0.5 g of benzoic acid, from 0.5 to 1.0 g ofphenol and from 0.1 to 0.5 g of an alkali fluoride and optionally other usual additives for the production of pharmaceutical preparations for the topical treatment of herpes virus effluorencences.

5. The use according to claim 4, characterized in that 100 g of the used product contains from 0.2 to 0.4 of the alkali fluoride.

6. The use according to claim 4, characterized in that the alkali fluoride is sodium fluoride.

## Claims for Contracting State AT

1. Process for the production of pharmaceutical products for the topical treatment of herpes-virus-effluorescences, characterizert in that bencoic acid, phenol and an alkali fluoride are dissolved in water or a mixture of water and alcohol in usual manners in such amounts that 100 g of the product contain from 0.1 to 0.5 g of benzoic acid, from 0.5 to 1.0 g of phenol and from 0.1 to 0.5 g of the alkali fluoride.

2. Process according to claim 1, characterized in that benzoid acid, phenol and the alkali fluoride are dissolved in such amounts that 100 g of the product contain from 0.2 to 0.4 g of benzoic acid, 0.6 to 0.8 g of phenol and 0.2 to 0.4 g of the alkali fluoride.

3. Process according to claim 1 or 2, characterized in that the alkali fluoride is sodium fluoride.

4. The use of an aqueous or aqueous-alcoholic solution containing per 100 g thereof from 0.1 to 0.5 g of benzoic acid, from 0.5 to 1.0 g ofphenol and from 0.1 to 0.5 g of an alkali fluoride and optionally other usual additives for the production of pharmaceutical preparations for the topical treatment of herpes virus effluorencences.

5. The use according to claim 4, characterized in that 100 g of the used product contains from 0.2 to 0.4 of the alkali fluoride.

6. The use according to claim 4, characterized in that the alkali fluoride is sodium fluoride.

## Revendications

1. Produit pharmaceutique contenant un mélange d'acide benzoïque, de phénol et d'un fluorure alcalin, en solution aqueuse ou hydroalcoolique, éventuellement en présence d'autres additifs classiques pour les préparations pharmaceutiques, pour le traitement topique des efflorescences d'herpès virus, caractérisé en ce que le produit contient pour 100 g 0,1 à 0,5 g d'acide benzoïque, 0,5 à 1,0 g de phénol et 0,1 à 0,5 g du fluorure alcalin.

2. Produit pharmaceutique selon la revendication 1, caractérisé en ce qu'il contient pour 100 g 0,2 à 0,4 g d'acide benzoïque, 0,6 à 0,8 g de phénol et 0,2 à 0,4 g du fluorure alcalin.

3. Produit pharmaceutique selon l'une des revendications 1 ou 2, caractérisé en ce que le fluorure alcalin est du fluorure de sodium.

4. Utilisation d'une solution aqueuse ou hydroalcoolique contenant pour 100 g 0,1 à 0,5 g d'acide benzoïque, 0,5 à 1,0 g de phénol et 0,1 à 0,5 g d'un fluorure alcalin et éventuellement d'autres additifs classiques, pour l'obtention de préparations pharmaceutiques pour le traitement topique des efflorescences d'herpès virus.

5. Utilisation selon la revendication 4, caractérisée en ce que le produit utilisé contient pour 100 g 0,2 à 0,4 g de fluorure alcalin.

6. Utilisation selon la revendication 4, caractérisée en ce que le fluorure alcalin est du fluorure de sodium.

## REVENDICATIONS POUR AT

1. Procédé pour l'obtention de produits pharmaceutiques pour le traitement topique d'efflorescences d'herpès virus, caractérisé en ce qu'on dissout de l'acide benzoïque, du phénol et un fluorure alcalin dans de l'eau ou dans un mélange d'eau et d'alcool, de manière classique, en des quantités telles que le produit contienne pour 100 g 0,1 à 0,5 g d'acide benzoïque, 0,5 à 1,0 g de phénol et 0,1 à 0,5 g du fluorure alcalin.

2. Procédé selon la revendication 1, caractérisé en ce qu'on dissout l'acide benzoïque, le phénol et le fluorure alcalin en des quantités telles que le produit résultant contienne pour 100 g 0,2 à 0,4 g d'acide benzoïque, 0,6 à 0,8 g de phénol et 0,2 à 0,4 g du fluorure alcalin.

3. Procédé selon l'une des revendications 1 ou 2, caractérisé en ce que le fluorure alcalin est du fluorure de sodium.

4. Utilisation d'une solution aqueuse ou hydroalcoolique contenant pour 100 g 0,1 à 0,5 g d'acide benzoïque, 0,5 à 1,0 g de phénol et 0,1 à 0,5 g d'un fluorure alcalin et éventuellement d'autres additifs classiques, pour l'obtention de préparations pharmaceutiques pour le traitement topique des efflorescences d'herpès virus.

5. Utilisation selon la revendication 4, caractérisée en ce que le produit utilisé contient pour 100 g 0,2 à 0,4 g de fluorure alcalin.

6. Utilisation selon la revendication 4, caractérisée en ce que le fluorure alcalin est du fluorure de sodium.